# EUROPEAN PATENT APPLICATION

(11) **EP 2 657 354 A1**
(43) Date of publication of application: **30.10.2013**
(21) Application number: 13178241.9
(22) Date of filing: 06.09.2010
(51) Int. Cl.: C12Q 1/68

(54) **Microrna expression signature in peripheral blood of patients affected by hepatocarcinoma or hepatic cirrhosis and uses thereof**

(30) Priority: 07.09.2009 IT MI20091538
(62) Divisional of application: 10771185.5
(71) Applicant: Istituto Nazionale Di Genetica Molecolare-INGM, 20122 Milano (IT)
(72) Inventor: Pagani, Massimiliano, 24069 Trescore Balneario (BG) (IT); De Francesco, Raffaele, 20146 Milano (IT); Abrignani, Sergio, 53040 Serre di Rapolano (SI) (IT); Rossetti, Grazisa, 20141 Milano (MI) (IT); Rossi, Riccardo Lorenzo, 24040 Stezzano (BG) (IT)
(74) Representative: Capasso, Olga

(57) **Abstract**

A method for diagnosing or prognosticating hepatocellular carcinoma, also in the early stages, or for assessing the risk of developing hepatocellular carcinoma, or for monitoring the effectiveness of an anti-tumour therapy against hepatocellular carcinoma by measuring the expression level of at least one miRNA gene product in a peripheral blood sample or in a biological fluid sample. Said method comprises measuring, in an isolated sample of peripheral blood or biological fluid, the expression level of at least one miRNA gene product, and comparing said measured expression level with a reference level. Such method can also be used to diagnose or assess the risk of developing liver cirrhosis in patients affected by chronic hepatitis, or to prognosticate the evolution of cirrhosis in patients affected by cirrhosis, or to monitor the effectiveness of a pharmacological therapy against liver cirrhosis.

SEQUENCE LISTING

<110>  ISTITUTO NAZIONALE DI GENETICA MOLECOLARE - INGM

<120>  MICRORNA EXPRESSION SIGNATURE IN PERIPHERAL BLOOD OF PATIENTS
       AFFECTED BY HEPATOCARCINOMA OR HEPATIC CIRRHOSIS AND USES THEREOF

<130>  21.I0197.12.IT.13

<160>  69

<170>  PatentIn version 3.3

<210>  1
<211>  22
<212>  RNA
<213>  Artificial

<220>
<223>  hsa-miR-223*

<400>  1
cguguauuug acaagcugag uu                                              22


<210>  2
<211>  23
<212>  RNA
<213>  Artificial

<220>
<223>  hsa-miR-19b-1*

<400>  2
aguuuugcag guuugcaucc agc                                             23


<210>  3
<211>  21
<212>  RNA
<213>  Artificial

<220>
<223>  hsa-miR-188-5p

<400>  3
caucccuugc augguggagg g                                               21


<210>  4
<211>  22
<212>  RNA
<213>  Artificial

<220>
<223>  hsa-miR-99b*

<400>  4
   caagcucgug ucuguggguc cg                                               22


<210>  5
<211>  22

<212>  RNA
<213>  Artificial

<220>
<223>  hsa-miR-509-3p

<400>  5
ugauugguac gucugugggu ag                                                22


<210>  6
<211>  20
<212>  RNA
<213>  Artificial

<220>
<223>  hsa-miR-760

<400>  6
cggcucuggg ucugugggga                                                   20


<210>  7
<211>  22
<212>  RNA
<213>  Artificial

<220>
<223>  hsa-miR-520c-3p

<400>  7
aaagugcuuc cuuuuagagg gu                                                22


<210>  8
<211>  24
<212>  RNA
<213>  Artificial

<220>
<223>  hsa-miR-573

<400>  8
cugaagugau guguaacuga ucag                                              24


<210>  9
<211>  20
<212>  RNA
<213>  Artificial

<220>
<223>  hsa-miR-877

<400>  9
guagaggaga uggcgcaggg                                                   20


<210>  10
<211>  22
<212>  RNA
<213>  Artificial

<220>
<223>  hsa-miR-643

<400>  10
acuuguaugc uagcucaggu ag                                                22


<210>  11
<211>  19
<212>  RNA
<213>  Artificial

<220>
<223>  hsa-miR-645

<400>  11
ucuaggcugg uacugcuga                                                    19


<210>  12
<211>  21
<212>  RNA
<213>  Artificial

<220>
<223>  hsa-miR-559

<400>  12
uaaaguaaau augcaccaaa a                                                 21


<210>  13
<211>  21
<212>  RNA
<213>  Artificial

<220>
<223>  hsa-miR-571

<400>  13
ugaguuggcc aucugaguga g                                                 21


<210>  14
<211>  22
<212>  RNA
<213>  Artificial

<220>
<223>  hsa-miR-122

<400>  14
uggaguguga caaugguguu ug                                                22


<210>  15
<211>  22
<212>  RNA
<213>  Artificial

<220>
<223>  hsa-miR-885-5p

<400>  15
uccauuacac uacccugccu cu                                              22


<210>  16
<211>  22
<212>  RNA
<213>  Artificial

<220>
<223>  hsa-miR-483-5p

<400>  16
aagacgggag gaaagaaggg ag                                              22


<210>  17
<211>  24
<212>  RNA
<213>  Artificial

<220>
<223>  hsa-miR-641

<400>  17
aaagacauag gauagaguca ccuc                                            24


<210>  18
<211>  22
<212>  RNA
<213>  Artificial

<220>
<223>  hsa-miR-375

<400>  18
uuuguucguu cggcucgcgu ga                                              22


<210>  19
<211>  23
<212>  RNA
<213>  Artificial

<220>
<223>  hsa-miR-567

<400>  19
aguauguucu uccaggacag aac                                             23


<210>  20
<211>  23
<212>  RNA
<213>  Artificial

<220>
<223>  hsa-miR-18b

<400>  20
uaaggugcau cuagugcagu uag                                             23

<210>  21
<211>  22
<212>  RNA
<213>  Artificial

<220>
<223>  hsa-miR-628-5p

<400>  21
augcugacau auuuacuaga gg                                                22


<210>  22
<211>  23
<212>  RNA
<213>  Artificial

<220>
<223>  hsa-miR-107

<400>  22
agcagcauug uacagggcua uca                                               23


<210>  23
<211>  21
<212>  RNA
<213>  Artificial

<220>
<223>  hsa-miR-23a

<400>  23
aucacauugc cagggauuuc c                                                 21


<210>  24
<211>  22
<212>  RNA
<213>  Artificial

<220>
<223>  hsa-miR-210

<400>  24
cugugcgugu gacagcggcu ga                                                22


<210>  25
<211>  22
<212>  RNA
<213>  Artificial

<220>
<223>  hsa-miR-1

<400>  25
uggaauguaa agaaguaugu au                                                22


<210>  26
<211>  22

<212>  RNA
<213>  Artificial

<220>
<223>  hsa-miR-377

<400>  26
aucacacaaa ggcaacuuuu gu                                                22


<210>  27
<211>  23
<212>  RNA
<213>  Artificial

<220>
<223>  hsa-miR-579

<400>  27
uucauuuggu auaaaccgcg auu                                               23


<210>  28
<211>  22
<212>  RNA
<213>  Artificial

<220>
<223>  hsa-miR-223

<400>  28
ugucaguuug ucaaauaccc ca                                                22


<210>  29
<211>  22
<212>  RNA
<213>  Artificial

<220>
<223>  hsa-miR-145*

<400>  29
ggauuccugg aaauacuguu cu                                                22


<210>  30
<211>  22
<212>  RNA
<213>  Artificial

<220>
<223>  hsa-miR-331-5p

<400>  30
cuagguaugg ucccagggau cc                                                22


<210>  31
<211>  22
<212>  RNA
<213>  Artificial

<220>
<223>  hsa-miR-140-5p

<400>  31
cagugguuuu acccuauggu ag                                                22


<210>  32
<211>  22
<212>  RNA
<213>  Artificial

<220>
<223>  hsa-miR-532-3p

<400>  32
ccucccacac ccaaggcuug ca                                                22


<210>  33
<211>  22
<212>  RNA
<213>  Artificial

<220>
<223>  hsa-miR-16

<400>  33
uagcagcacg uaaauauugg cg                                                22


<210>  34
<211>  22
<212>  RNA
<213>  Artificial

<220>
<223>  hsa-miR-494

<400>  34
ugaaacauac acgggaaacc uc                                                22


<210>  35
<211>  22
<212>  RNA
<213>  Artificial

<220>
<223>  hsa-miR-132

<400>  35
uaacagucua cagccauggu cg                                                22


<210>  36
<211>  22
<212>  RNA
<213>  Artificial

<220>
<223>  hsa-miR-29a

<400>  36
uagcaccauc ugaaaucggu ua                                              22


<210>  37
<211>  22
<212>  RNA
<213>  Artificial

<220>
<223>  hsa-miR-590-5p

<400>  37
gagcuuauuc auaaaagugc ag                                              22


<210>  38
<211>  21
<212>  RNA
<213>  Artificial

<220>
<223>  hsa-miR-195

<400>  38
uagcagcaca gaaauauugg c                                               21


<210>  39
<211>  22
<212>  RNA
<213>  Artificial

<220>
<223>  hsa-miR-197

<400>  39
uucaccaccu ucuccaccca gc                                              22


<210>  40
<211>  22
<212>  RNA
<213>  Artificial

<220>
<223>  hsa-miR-193a-5p

<400>  40
ugggucuuug cgggcgagau ga                                              22


<210>  41
<211>  22
<212>  RNA
<213>  Artificial

<220>
<223>  hsa-miR-24

<400>  41
uggcucaguu cagcaggaac ag                                              22

<210>  42
<211>  21
<212>  RNA
<213>  Artificial

<220>
<223>  hsa-miR-140-3p

<400>  42
uaccacaggg uagaaccacg g                                                21


<210>  43
<211>  23
<212>  RNA
<213>  Artificial

<220>
<223>  hsa-miR-19b

<400>  43
ugugcaaauc caugcaaaac uga                                              23


<210>  44
<211>  23
<212>  RNA
<213>  Artificial

<220>
<223>  hsa-miR-19a

<400>  44
ugugcaaauc uaugcaaaac uga                                              23


<210>  45
<211>  21
<212>  RNA
<213>  Artificial

<220>
<223>  hsa-miR-222

<400>  45
agcuacaucu ggcuacuggg u                                                21


<210>  46
<211>  23
<212>  RNA
<213>  Artificial

<220>
<223>  hsa-miR-93

<400>  46
caaagugcug uucgugcagg uag                                              23


<210>  47
<211>  22

<212>  RNA
<213>  Artificial

<220>
<223>  hsa-miR-15b

<400>  47
uagcagcaca ucaugguuua ca                                                22


<210>  48
<211>  22
<212>  RNA
<213>  Artificial

<220>
<223>  hsa-miR-25

<400>  48
cauugcacuu gucucggucu ga                                                22


<210>  49
<211>  22
<212>  RNA
<213>  Artificial

<220>
<223>  hsa-miR-655

<400>  49
auaauacaug guuaaccucu uu                                                22


<210>  50
<211>  22
<212>  RNA
<213>  Artificial

<220>
<223>  hsa-miR-365

<400>  50
uaaugccccu aaaaauccuu au                                                22


<210>  51
<211>  22
<212>  RNA
<213>  Artificial

<220>
<223>  hsa-miR-199a-3p

<400>  51
acaguagucu gcacauuggu ua                                                22


<210>  52
<211>  22
<212>  RNA
<213>  Artificial

<220>
<223>  hsa-miR-625*

<400>  52
gacuauagaa cuuucccccu ca                                                22


<210>  53
<211>  23
<212>  RNA
<213>  Artificial

<220>
<223>  hsa-miR-142-3p

<400>  53
uguaguguuu ccuacuuuau gga                                               23


<210>  54
<211>  23
<212>  RNA
<213>  Artificial

<220>
<223>  hsa-miR-454

<400>  54
uagugcaaua uugcuuauag ggu                                               23


<210>  55
<211>  22
<212>  RNA
<213>  Artificial

<220>
<223>  hsa-miR-345

<400>  55
gcugacuccu aguccagggc uc                                                22


<210>  56
<211>  22
<212>  RNA
<213>  Artificial

<220>
<223>  hsa-let-7b

<400>  56
ugagguagua gguugugugg uu                                                22


<210>  57
<211>  23
<212>  RNA
<213>  Artificial

<220>
<223>  hsa-miR-20a

<400>  57
uaaagugcuu auagugcagg uag                                              23


<210>  58
<211>  22
<212>  RNA
<213>  Artificial

<220>
<223>  hsa-miR-186

<400>  58
caaagaauuc uccuuuuggg cu                                               22


<210>  59
<211>  23
<212>  RNA
<213>  Artificial

<220>
<223>  hsa-miR-191

<400>  59
caacggaauc ccaaaagcag cug                                              23


<210>  60
<211>  22
<212>  RNA
<213>  Artificial

<220>
<223>  hsa-miR-451

<400>  60
aaaccguuac cauuacugag uu                                               22


<210>  61
<211>  22
<212>  RNA
<213>  Artificial

<220>
<223>  hsa-let-7g

<400>  61
ugagguagua guuuguacag uu                                               22


<210>  62
<211>  22
<212>  RNA
<213>  Artificial

<220>
<223>  hsa-miR-93*

<400>  62
acugcugagc uagcacuucc cg                                               22

<210>  63
<211>  22
<212>  RNA
<213>  Artificial

<220>
<223>  hsa-miR-138-1*

<400>  63
gcuacuucac aacaccaggg cc                                                22


<210>  64
<211>  22
<212>  RNA
<213>  Artificial

<220>
<223>  hsa-miR-485-3p

<400>  64
gucauacacg gcucuccucu cu                                                22


<210>  65
<211>  22
<212>  RNA
<213>  Artificial

<220>
<223>  hsa-miR-299-3p

<400>  65
uaugugggau gguaaaccgc uu                                                22


<210>  66
<211>  22
<212>  RNA
<213>  Artificial

<220>
<223>  hsa-miR-133b

<400>  66
   uuuggucccc uucaaccagc ua                                                22


<210>  67
<211>  22
<212>  RNA
<213>  Artificial

<220>
<223>  hsa-miR-519d

<400>  67
caaagugccu cccuuuagag ug                                                22


<210>  68
<211>  21

<212>  RNA
<213>  Artificial

<220>
<223>  hsa-miR-190b

<400>  68
ugauauguuu gauauugggu u                                                21


<210>  69
<211>  20
<212>  RNA
<213>  Artificial

<220>
<223>  hsa-miR-599

<400>  69
guugugucag uuuaucaaac                                                  20

## Description

The present invention relates to a method for diagnosing or prognosticating hepatocellular carcinoma, also in the early stages, or for assessing the risk of developing hepatocellular carcinoma or for monitoring the effectiveness of an anti-tumour therapy against hepatocellular carcinoma.

Hepatocellular carcinoma is one of the most aggressive, common neoplasias in the world, characterized by an often unfavourable course. The main therapies to which recourse is made in cases of hepatocellular carcinoma involve surgical resection or a liver transplant. However, the low postoperative survival rate (30-40% after five years) and frequent post-surgery reappearance of metastasis in patients undergoing a surgical resection treatment considerably complicate the clinical approach toward hepatocellular carcinoma. This limit is further exacerbated by the reduced possibility of surgical treatment, which is in fact restricted to only a small percentage of patients (around 20% of patients with hepatocellular carcinoma), in particular those patients found to have small lesions and relatively normal hepatic parameters.

Liver transplant as an effective therapeutic solution for hepatocellular carcinoma is still today a highly debated issue and the opinions on this topic are controversial given the low availability of organs, and above all the difficulty of classifying, and identifying patients suited for treatment, mainly when the pathology is in the initial stages. Though well-codified, generally accepted methods for classifying the pathological progression of many forms of tumours are available today, hepatocellular carcinoma represents an exception. The clinical classification of hepatocellular carcinoma and the correlated therapeutic indications entail very complex procedures and depend both on the degree of tumour progression and residual liver function. There exist various classification and clinical staging systems for hepatocellular carcinoma, but the scientific community has not yet given an opinion as to the most appropriate and effective method. Therefore, the identification of specific prognostic markers capable of providing new classification and staging criteria applicable to patients affected by hepatocellular carcinoma represents an indispensable objective, especially because the choice of the therapeutic treatment best suited for the patient depends on it.

The objective of a universally accepted staging is potentially useful for improving the accuracy of the prognosis in individual patients, favouring the selection of patients for different therapies and, finally, adapting groups of patients based on therapeutic efficacy.

The identification of molecular biomarkers could offer hope of improving the diagnosis or prognosis of hepatocellular carcinoma, assessing the risk of developing hepatocellular carcinoma and monitoring the effectiveness of a therapeutic treatment against hepatocellular carcinoma.

In this context, the technical problem at the basis of the present invention is to provide a method for the classification and staging of hepatocellular carcinoma which is not invasive, is simple and fast, but at the same time accurate and reproducible, and which can fulfill the need for a "universal" classification and staging, useful for assuring the choice of the best therapeutic treatment for each individual patient. In the context of the present invention, the term "non- invasive" signifies the possibility, by means of a simple blood test, of devising made-to-measure treatments for individual patients, as opposed to relying on disadvantageous methods with costly imaging and invasive biopsies, which at present represent the classic clinical approach for cancer diagnosis, prognosis and hence therapy. In particular, a specific panel of biomarkers, present and stable in the bloodstream, can be used as a molecular "fingerprint" of hepatocellular carcinoma.

Such technical problem is resolved by a method for diagnosing or prognosticating hepatocellular carcinoma, also in the early stages, for assessing the risk of developing hepatocellular carcinoma or for monitoring the effectiveness of an anti-tumour therapy against hepatocellular carcinoma, as described in the appended claims.

The present invention relates to a method for diagnosing or prognosticating hepatocellular carcinoma, also in the early stages, for assessing the risk of developing hepatocellular carcinoma or for monitoring the effectiveness of an anti-tumour therapy against hepatocellular carcinoma, which comprises measuring, preferably by quantitative RT-PCR, the expression level of at least one microRNA (miRNA) gene product in a peripheral blood sample or in a biological fluid sample, and comparing said measured expression level with a reference level. An alteration in the expression levels of a miRNA gene product in a sample of the test subject, as compared to a control sample, is indicative of the fact that the subject is affected by hepatocellular carcinoma or has an increased risk of developing hepatocellular carcinoma. Furthermore, an alteration in the expression levels of a miRNA gene product in a sample of the test subject, as compared to a control sample, is indicative of the effectiveness, evolution and outcome of a therapy against hepatocellular carcinoma.

An alteration in the expression levels of a miRNA gene product in a sample of the test subject, as compared to a control sample, is also indicative of the evolution of the disease and hence of the prognosis thereof.

Such method can also be used to diagnose or assess the risk of developing liver cirrhosis in patients affected, for example, by chronic hepatitis or in healthy subjects, or to prognosticate the evolution of cirrhosis in patients affected by cirrhosis, or to monitor the effectiveness of a pharmacological therapy against liver cirrhosis.

In this case the method comprises measuring, preferably by quantitative RT-PCR, the expression level of at least one microRNA (miRNA) gene product in a peripheral blood sample or else in a biological fluid sample, and comparing said measured expression level with a reference level. An alteration in the expression levels of a miRNA gene product in a sample of the test subject, as compared to a control sample, is indicative of the fact that the subject is affected by liver cirrhosis or has an increased risk of developing liver cirrhosis, as for example in the case of patients affected by chronic hepatitis. Such alteration is also indicative of the effectiveness, evolution and outcome of a therapy against liver cirrhosis.

Said alteration in levels is also indicative of the evolution of the disease and hence of the prognosis thereof.

It is an object of the invention a method for diagnosing or prognosticating hepatocellular carcinoma, also in the early stages, or for assessing the risk of developing hepatocellular carcinoma, or for monitoring the effectiveness of an anti-tumour therapy against hepatocellular carcinoma, comprising the step of measuring, in an isolated sample of peripheral blood or biological fluid, the overexpression of at least one miRNA gene product chosen from the group consisting of SEQ ID NO 1-19, SEQ ID NO 63-66 and SEQ ID 69, and the underexpression of at least one miRNA gene product chosen from the group consisting of SEQ ID NO 20-62 and SEQ ID NO 67-68, as compared to a reference expression level.

Preferably a measurement is made of the overexpression of at least one miRNA gene product chosen from the group consisting of SEQ ID NO 6, 9, 12, 13, 14, 15, 16 and 65, and the underexpression of at least one miRNA gene product chosen from the group consisting of SEQ ID NO 23, 28, 30, 31, 33, 36, 37, 39, 40, 41 and 67, as compared to a reference expression level. 3. Still preferably said peripheral blood sample is chosen from among whole blood, blood peripheral mononucleated cells, serum or plasma; said biological fluid sample is chosen between urine or saliva.

Still preferably said method for monitoring the effectiveness of an anti-tumour therapeutic treatment comprises measuring the alteration in the expression levels of at least one miRNA gene product in a sample of the test subject, as compared to a sample of the same subject in different phases of the anti-tumour therapeutic treatment.

Yet preferably said method for monitoring the effectiveness of an anti-tumour therapeutic treatment comprises comparing peripheral blood samples from patients affected by hepatocellular carcinoma who are undergoing an anti- tumour therapeutic treatment and samples from patients affected by hepatocellular carcinoma who are not undergoing an anti-tumour therapeutic treatment and measuring the alteration in the expression levels of a miRNA gene product between the two groups of patients.

Yet preferably said method is a method for diagnosing or assessing the risk of developing liver cirrhosis, or for prognosticating the evolution of liver cirrhosis in patients affected by cirrhosis, or for monitoring the effectiveness of a pharmacological therapy against liver cirrhosis.

It is a further object of the invention the use of the method as defined above to identify new therapeutic targets.

It is a further object of the invention a pharmaceutical composition comprising a pharmaceutically acceptable carrier and at least one isolated miRNA gene product as defined above and/or a nucleic acid complementary thereto.

Preferably the composition is for use in the treatment of hepatocellular carcinoma or for the treatment of liver cirrhosis.

Further characteristics and advantages of the method according to the present invention will become more apparent from the experimental results illustrated in the appended figures, in which:

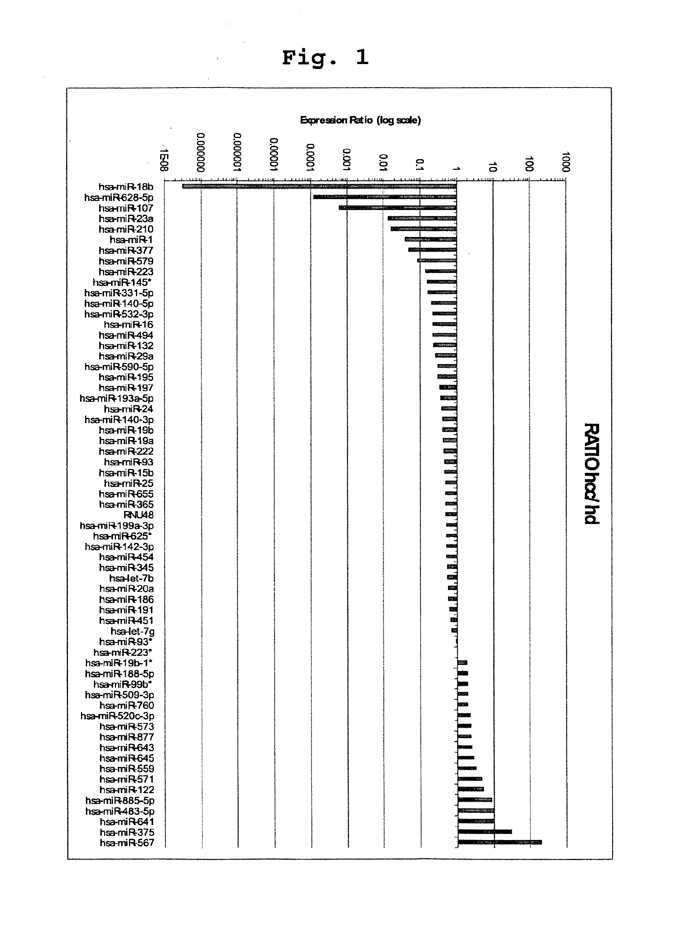

- Figure 1 shows a graph of the values of the relative expression ratio between blood samples from subjects affected by hepatocellular carcinoma (HCC) and blood samples from healthy subjects (HD-control) ; values between 0 and 1 indicate overexpressed miRNAs in the samples from healthy subjects, whereas values greater than 1 refer to overexpressed miRNAs in the samples from subjects affected by hepatocellular carcinoma;

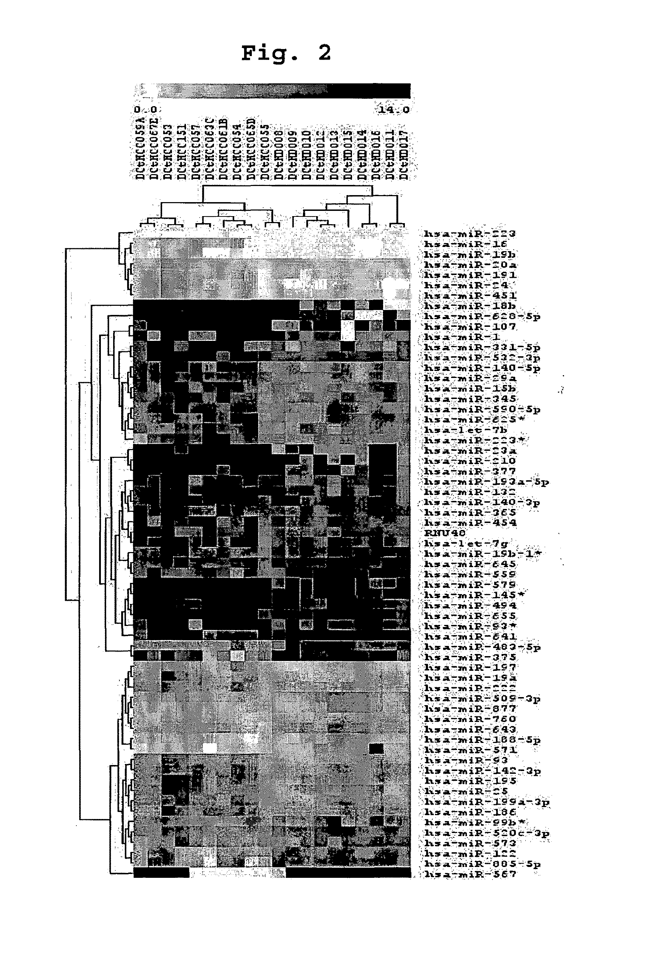

- Figure 2 shows a graphical representation, by colour gradient (heatmap), of the ΔCt values (Ct: Cycle threshold) for the 62 miRNAs differentially expressed, in a significant manner according to the t-test analysis, between unpaired samples, 10 from healthy subjects and 10 from subjects with hepatocellular carcinoma HCC (p-value <0.05);

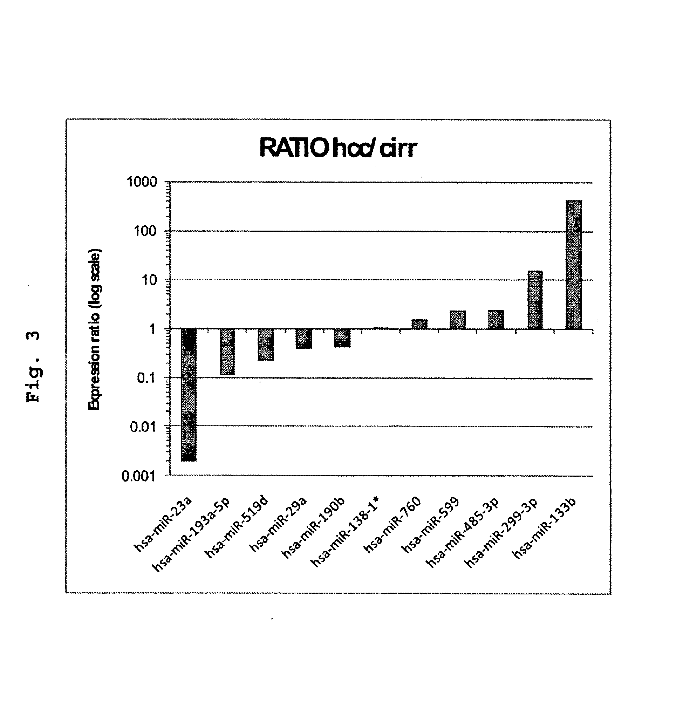

- Figure 3 shows a graph of the values of the relative expression ratio between blood samples from subjects affected by liver cirrhosis and blood samples from the same cirrhotic subjects who subsequently developed hepatocellular carcinoma; values between 0 and 1 indicate overexpressed miRNAs in the samples from cirrhotic subjects, whereas values greater than 1 refer to overexpressed miRNAs in the samples from subjects with hepatocellular carcinoma;

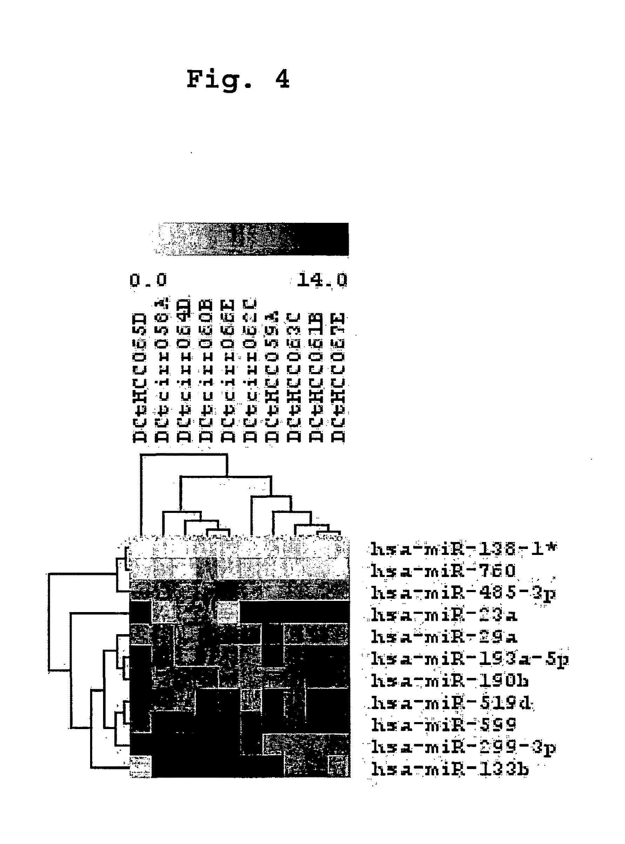

- Figure 4 shows a graphical representation, by colour gradient (heatmap), of the ΔCt values for the 11 miRNAs differentially expressed, in a significant manner according to the paired t-test analysis, between 5 blood samples from subjects affected by liver cirrhosis and 5 blood samples from the same cirrhotic subjects who subsequently developed hepatocellular carcinoma.

MiRNAs are molecules naturally- present in many organisms, including animals, plants and viruses, and play a fundamental role in the control of gene expression by regulating, in a specific manner, the stability and translation of messenger RNAs (mRNAs).

MiRNAs are initially expressed as long precursor RNA molecules, or pri-miRNAs, which by means of a complex mechanism of nucleocytoplasmic processing, are transformed into the mature form (miRNA), characterized by a length of 17-24 nucleotides. The function of many miRNAs is not known; however, various studies have demonstrated the key role that miRNAs have in gene regulation in many fundamental biological functions such as apoptosis, haematopoietic development and cellular differentiation.

The biological and clinical relevance of miRNA expression profiles has been demonstrated in solid human tumours (like breast tumours) and chronic lymphatic leukaemia.

A further property of miRNAs is their presence, in a stable, resistant RNA form, in blood (serum and plasma) and in various other biological fluids; it has recently been demonstrated that the blood of patients affected by prostate carcinoma or ovarian cancer shows peculiar miRNA expression profiles. For the purposes of the present invention, the at least one miRNA gene product used in the method is at least one miRNA.

The at least one miRNA gene product is chosen, individually or in combination, from the group consisting of SEQ ID NO 1-69.

In a preferred embodiment of the invention, the at least one miRNA gene product is selected, individually or in combination, from the group consisting of SEQ ID NO 1-19 and SEQ ID NO 63-66 and SEQ ID 69; more preferably it is chosen from the group consisting of: SEQ ID NO 6, SEQ ID NO 9, SEQ ID NO 12, SEQ ID NO 13, SEQ ID NO 14, SEQ ID NO 15, SEQ ID NO 16 and SEQ ID NO 65. Such miRNA sequences are characterized by a higher relative expression level in a sample of a subject affected by hepatocellular carcinoma as compared to a control, who may be a healthy or cirrhotic subject.

In another preferred embodiment of the present invention, the at least one miRNA gene product is selected, individually or in combination, from the group consisting of SEQ. ID NO 20-62 and SEQ. ID NO 67-68; more preferably it is chosen from the group consisting of:, SEQ ID NO 23, SEQ ID NO 28, SEQ ID NO 30, SEQ ID NO 31, SEQ ID NO 33, SEQ ID NO 36, SEQ ID NO 37, SEQ ID NO 39, SEQ ID NO 40, SEQ ID NO 41 and SEQ ID NO 67.

Such miRNA sequences are characterized by a lower relative expression level in samples from subjects affected by hepatocellular carcinoma as compared to a control, who may be a healthy or cirrhotic subject.

In a preferred embodiment of the present invention the at least one miRNA gene product is selected, individually or in combination, from among the sequences: SEQ ID NO 6 , 9, 12, 13, 14, 15, 16, 23, 28, 30, 31, 33, 36, 37, 39, 40, 41, 65 and 67. The method, which is the subject matter of the present invention, is preferably carried out in vitro, in particular on blood samples or biological fluid of a human subject.

The peripheral blood sample to be investigated can be whole blood, or isolated (ex vivo) peripheral blood mononucleated cells, serum or plasma.

The sample to be investigated can also be any biological fluid, for example urine or saliva. The method described relates to hepatocellular carcinoma, in an advanced or even early stage, in particular to trabecular, pseudoglandular, compact, or scirrhous hepatocellular carcinoma, with different degrees of differentiation. The method of the invention is used to diagnose whether a subject is affected by hepatocellular carcinoma or is at risk of developing said pathology by verifying the presence of any alteration in the expression levels of a miRNA gene product in a peripheral blood or biological fluid sample of the test subject, as compared to a control sample.

The method of the invention is also used to define the prognosis of hepatocellular carcinoma by comparing the expression levels of at least one miRNA gene product in a peripheral blood or biological fluid sample of a subject affected by hepatocellular carcinoma and a reference level. An alteration in the expression levels of the at least one miRNA gene product in a sample of the test subject, as compared to a reference sample, is indicative of the degree of tumour advancement, from which it is possible to deduce the disease prognosis. The method of the invention is also used to monitor the effectiveness of a therapeutic anti-tumour treatment, in particular a chemo/radiotherapy treatment. In this case the method comprises comparing the expression levels of at least one miRNA gene product in a peripheral blood or biological fluid sample of the test subject with a reference sample. An alteration in the expression levels of the at least one miRNA gene product in a sample of the test subject, as compared to a sample of the same subject in different phases of the therapeutic treatment in question, is indicative of the effectiveness of the treatment itself.

Alternatively, the method for determining the effectiveness of a therapeutic anti-tumour treatment comprises comparing peripheral blood samples from patients affected by hepatocellular carcinoma who are undergoing therapeutic anti-tumour treatment and samples from patients affected by hepatocellular carcinoma who are not undergoing therapeutic anti-tumour treatment. An alteration in the expression levels of a miRNA gene product between the two groups of patients is indicative of whether a new method of therapeutic anti-tumour treatment is valid and effective or not. In an alternative embodiment, the method of the invention can also be used to diagnose or assess the risk of developing liver cirrhosis, for example in patients affected by chronic hepatitis or in healthy patients, or to prognosticate the evolution of cirrhosis in patients affected by cirrhosis, or to monitor the effectiveness of a pharmacological therapy against liver cirrhosis.

In this case the method comprises measuring, preferably by quantitative RT-PCR, the expression level of at least one microRNA (miRNA) gene product in a peripheral blood sample or in a biological fluid sample, and comparing said measured expression level with a reference level. An alteration in the expression levels of a miRNA gene product in a sample of the test subject, as compared to a control sample, is indicative of the fact that the subject is affected by liver cirrhosis or has an increased risk of developing liver cirrhosis, as for example in the case of patients affected by chronic hepatitis.

Such alteration is also indicative of the effectiveness, evolution and outcome of a therapy against liver cirrhosis.

Such alteration in levels is also indicative of the evolution of the disease and hence the prognosis thereof.

In this embodiment the miRNA gene product is as specified previously.

In another embodiment, the method according to the present invention can also be used in combination with other diagnostic/prognostic methods presently in use, as a valid complement to said investigative techniques.

For example, the method can be applied in combination with: microarrays, proteomic and immunological analyses, and sequencing analyses of specific DNA sequences for the purpose of defining an ad hoc therapeutic approach for individual patients. Completing the clinical information derived from known investigative techniques with that of the present invention would help to address the treatment of a patient affected by hepatocellular carcinoma or cirrhosis in a completely personalised manner that is advantageous as regards both the diagnosis and the prognosis and therapy.

In another embodiment, the method of the invention can be used to identify new therapeutic targets.

Each miRNA has the capability of regulating the expression of hundreds of genes and can thus modulate the activity of many molecular signal transduction pathways inside the cell. Therefore, the miRNA panels identified in the peripheral blood of a subject affected by tumour reflect the biology of the primary tumour.

Said miRNAs are useful as biomarkers for identifying the pathology, defining the response to therapies and monitoring any possible recurrences of the hepatocellular carcinoma. Such miRNAs are also useful for defining the altered molecular pathways in hepatocellular carcinoma and can contribute, therefore, to identifying new therapeutic targets.

The present invention also relates to a pharmaceutical composition for treating hepatocellular carcinoma or liver cirrhosis, comprising a pharmaceutically acceptable carrier and at least one isolated miRNA gene product and/or a nucleic acid complementary thereto, which is up- or down- regulated in the peripheral blood of a subject affected by hepatocellular carcinoma or liver cirrhosis, as compared to a suitable control sample. The at least one isolated miRNA gene product is chosen, individually or in different combinations, from among the sequences previously identified.

The present invention further relates to a method for identifying an anti-hepatocellular carcinoma or anti- liver cirrhosis agent which comprises a step of administering a test substance to isolated cells (ex- vivo). After administration, a measurement is made of the level of at least one miRNA gene product whose increased expression is associated with hepatocellular carcinoma or liver cirrhosis.

Subsequently, the expression level of said at least one miRNA gene product in the treated cells is compared with that in the control cells. A decrease in said expression level is indicative of the fact that the test substance is an anti-hepatocellular carcinoma or anti- liver cirrhosis agent.

### EXPERIMENTAL PART

### EXAMPLE 1

The investigations were carried out on ten subjects affected by hepatocellular carcinoma. The tissue analysed consisted in peripheral blood and the experimental control was represented by the peripheral blood of ten subjects free of symptoms or traces of tumour.

The total RNA was extracted using the mirVanaTM miRNA Isolation Kit (Cat# AM1561- Ambion). Synthetic RNA ath-miR159a, Arabidopsis thaliana microRNA not expressed in man, was added as a quantitative normalizer (3 fmoles per aliquot of serum). An aliquot of the sample (3 µL of the total 50 µL of extracted RNA) was submitted to the reverse transcription reaction conducted using the TaqMan^{®} MicroRNA Reverse Transcription kit in the presence of a solution of MgCl₂ 5 mM (Part no. 4366597 - Applied Biosystems). MegaplexTM RT Primers were used as primers for the reverse transcription, a set of 2 predefined pools (Pool A and Pool B) of 380 RT primers each, which permits the simultaneous synthesis of cDNAs from mature miRNAs (MegaplexTM RT Primers Human Pool A, Part No.: 4399966; Human Pool B, Part No.: 4399968 - Applied Biosystems). Final reaction volume (µL): 7.5.

### Incubation conditions for a reaction cycle:

16 °C 2 min
42 °C 1 min
50 °C 1 sec
85 °C 5 min
4 °C ∞
(for 40 cycles)

The cDNA thus produced was pre-amplified (2.5 µL of the 7.5 µL) using TaqMan PreAmp Master Mix (2x) (Part No.: 4384266 - Applied Biosystems) and MegaplexTM PreAmp Primers, a set of 2 pools of gene-specific primers, forward and reverse (Megaplex(TM) PreAmp Primers, Human Pool A, Part no. 4399233; Human Pool B (Part no. 4399201 - Applied Biosystems).

Final reaction volume (µL): 25.

### Incubation conditions:

95 °C 10 min
55 °C 2 min
72 °C 2 min
95 °C 15 sec
60 °C 4 min x 12 cycles
4 °C ∞

The pre-amplified cDNA was used for the real-time PCR reaction. The reaction was conducted using TaqMan Universal PCR Master Mix, No Amperase UNG, 2X (Part No: 4326614 - Applied Biosystems) in 900 final µL, loaded into 2 sets of microfluidic cards, TaqMan^{®} Human MicroRNA Low Density Arrays (Part No.: 4400238 - Applied Biosystems), with 384 wells each, containing TaqMan probes. Such analysis (Array A and Array B) enables the quantification of the gene expression levels of 665 miRNAs and of the related controls (http://www3.appliedbiosystems.com/cms/groups/portal/documents/g eneraldocuments/cms_052133.xls).

The internal control ath-miR159a can be used to calculate the relative gene expression. The relative expression of each miRNA can be calculated using the equation 2^{-ΔCt}, where ΔCt = CtmiRNA - Ctath-miR159a.

The relative expression of each miRNA calculated by means of PCR can be analysed using statistical methods such as the t-test or ANOVA. The ΔCt data are submitted to "hierarchical clustering" analysis and the corresponding results are displayed in a graphical "heatmap" (see Fig. 2).

The "quantitative RT PCR" analysis showed the presence of 62 miRNAs, listed in Table 1, which are present in higher or lower quantities in subjects with hepatocellular carcinoma versus the controls.

**Table 1:**

| **miRNA** | **raw p-val** | **higher in** | **Target Sequence** | **miRNA Sequence Number** |
|---|---|---|---|---|
| hsa-miR-223 | 1.69E-06 | HD | UGUCAGUUUGUCAAAUACCCCA | SEQ ID NO 28 |
| hsa-miR-483-Sp | 3.17E-05 | HCC | AAGACGGGAGGAAAGAAGGGAG | SEQ ID NO 16 |
| hsa-miR-16 | 3.75E-05 | HD | UAGCAGCACGUAAAUAUUGGCG | SEQ ID NO 33 |
| hsa-miR-122 | 2.41E-04 | HCC | UGGAGUGUGACAAUGGUGUUUG | SEQ ID NO 14 |
| hsa-miR-29° | 2.93E-04 | HD | UAGCACCAUCUGAAAUCGGUUA | SEQ ID NO 36 |
| hsa-miR-140-5p | 3.73E-04 | HD | CAGUGGUUUUACCCUAUGGUAG | SEQ ID NO 31 |
| hsa-miR-532-3p | 3.98E-04 | HD | CCUCCCACACCCAAGGCUUGCA | SEQ ID NO 32 |
| hsa-miR-590-5p | 7.41E-04 | HD | GAGCUUAUUCAUAAAAGUGCAG | SEQ ID NO 37 |
| hsa-miR-24 | 0.001326056 | HD | UGGCUCAGUUCAGCAGGAACAG | SEQ ID NO 41 |
| hsa-miR-195 | 0.001366361 | HD | UAGCAGCACAGAAAUAUUGGC | SEQ ID NO 38 |
| hsa-miR-197 | 0.001388423 | HD | UUCACCACCUUCUCCACCCAGC | SEQ ID NO 39 |
| hsa-miR-643 | 0.002119931 | HCC | ACUUGUAUGCUAGCUCAGGUAG | SEQ ID NO 10 |
| hsa-miR-93 | 0.002347399 | HD | CAAAGUGCGUUCGUGCAGGUAG | SEQ ID NO 46 |
| hsa-miR-222 | 0.00249573 | HD | AGCUACAUCUGGCUACUGGGU | SEQ ID NO 45 |
| hsa-miR-571 | 0.002797201 | HCC | UGAGUUGGCCAUCUGAGUGAG | SEQ ID NO 13 |
| hsa-miR-23° | 0.002827722 | HD | AUCACAUUGCCAGGGAUUUCC | SEQ ID NO 23 |
| hsa-miR-877 | 0.003071473 | HCC | GUAGAGGAGAUGGCGCAGGG | SEQ ID NO 9 |
| hsa-miR-19b | 0.003224636 | HD | UGUGCAAAUCCAUGCAAAACUGA | SEQ ID NO 43 |
| hsa-miR-132 | 0.003554063 | HD | UAACAGUCUACAGCCAUGGUCG | SEQ ID NO 35 |
| hsa-miR-520c-3p | 0.004731762 | HCC | AAAGUGCUUCCUUUUAGAGGGU | SEQ ID NO 7 |
| hsa-miR-19° | 0.005161863 | HD | UGUGCAAAUCUAUGCAAAACUGA | SEQ ID NO 44 |
| hsa-miR-885-5p | 0.005185759 | HCC | UCCAUUACACUACCCUGCCUCU | SEQ ID NO 15 |
| hsa-miR-188-5p | 0.005383855 | HCC | CAUCCCUUGCAUGGUGGAGGG | SEQ ID NO 3 |
| hsa-miR-494 | 0.005799115 | HD | UGAAACAUACACGGGAAACCUC | SEQ ID NO 34 |
| hsa-miR-559 | 0.00580249 | HCC | UAAAGUAAAUAUGCACCAAAA | SEQ ID NO 12 |
| hsa-miR-331-5p | 0.007425596 | HD | CUAGGUAUGGUCCCAGGGAUCC | SEQ ID NO 30 |
| hsa-miR-142-3p | 0.007429025 | HD | UGUAGUGUUUCCUACUUUAUGGA | SEQ ID NO 53 |
| hsa-miR-15b | 0.010680858 | HD | UAGCAGCACAUCAUGGUUUACA | SEQ ID NO 47 |
| hsa-miR-140-3p | 0.011537802 | HD | UACCACAGGGUAGAACCACGG | SEQ ID NO 42 |
| hsa-miR-25 | 0.012526199 | HD | CAUUGCACUUGUCUCGGUCUGA | SEQ ID NO 48 |
| hsa-miR-760 | 0.012662118 | HCC | CGGCUCUGGGUCUGUGGGGA | SEQ ID NO 6 |
| hsa-miR-199°-3p | 0.014095253 | HD | ACAGUAGUCUGCACAUUGGUUA | SEQ ID NO 51 |
| hsa-miR-655 | 0.014120932 | HD | AUAAUACAUGGUUAACCUCUUU | SEQ ID NO 49 |
| hsa-miR-567 | 0.014409184 | HCC | AGUAUGUUCUUCCAGGACAGAAC | SEQ ID NO 19 |
| hsa-miR-345 | 0.014628208 | HD | GCUGACUCCUAGUCCAGGGCUC | SEQ ID NO 55 |
| hsa-miR-20° | 0.015044568 | HD | UAAGUGCUUAUAGUGCAGGUAG | SEQ ID NO 57 |
| hsa-miR-365 | 0.015725587 | HD | UAAUGCCCCUAAAAAUCCUUAU | SEQ ID NO 50 |
| hsa-miR-628-5p | 0.01646383 | HD | AUGCUGACAUAUUUACUAGAGG | SEQ ID NO 21 |
| hsa-miR-509-3p | 0.017252065 | HCC | UGAUUGGUACGUCUGUGGGUAG | SEQ ID NO 5 |
| hsa-miR-579 | 0.017330982 | HD | UUCAUUUGGUAUAAACCGCGAUU | SEQ ID NO 27 |
| hsa-miR-223* | 0.01885562 | HCC | CGUGUAUUUGACAAGCUGAGUU | SEQ ID NO 1 |
| hsa-miR-99b* | 0.022084232 | HCC | CAAGCUCGUGUCUGUGGGUCCG | SEQ ID NO 4 |
| hsa-miR-145* | 0.023664307 | HD | GGAUUCCUGGAAAUACUGUUCU | SEQ ID NO 29 |
| hsa-miR-107 | 0.023735054 | HD | AGCAGCAUUGUACAGGGCUAUCA | SEQ ID NO 22 |
| hsa-miR-1 | 0.02399486 | HD | UGGAAUGUAAAGAAGUAUGUAU | SEQ ID NO 25 |
| hsa-miR-377 | 0.026372321 | HD | AUCACACAAAGGCAACUUUUGU | SEQ ID NO 26 |
| hsa-miR-454 | 0.02798928 | HD | UAGUGCAAUAUUGCUUAUAGGGU | SEQ ID NO 54 |
| hsa-let-7g | 0.029252954 | HD | UGAGGUAGUAGUUUGUACAGUU | SEQ ID NO 61 |
| hsa-miR-193°-5p | 0.029725004 | HD | UGGGUCUUUGCGGGCGAGAUGA | SEQ ID NO 40 |
| hsa-miR-451 | 0.03039734 | HD | AAACCGUUACCAUUACUGAGUU | SEQ ID NO 60 |
| hsa-let-7b | 0.031215172 | HD | UGAAGGUAGUAGGUUGUGUGGUU | SEQ ID NO 56 |
| hsa-miR-625* | 0.0320151 | HD | GACUAUAGAACUUUCCCCCUCA | SEQ ID NO 52 |
| hsa-miR-191 | 0.035316132 | HD | CAACGGAAUCCCAAAAGCAGCUG | SEQ ID NO 59 |
| hsa-miR-18b | 0.036119234 | HD | UAAGGUGCAUCUAGUGCAGUUAG | SEQ ID NO 20 |
| hsa-miR-375 | 0.036783654 | HCC | UUUGUUCGUUCGGCUCGCGUGA | SEQ ID NO 18 |
| hsa-miR-210 | 0.038199093 | HD | CUGUGCGUGUGACAGCGGCUGA | SEQ ID NO 24 |
| hsa-miR-573 | 0.038235087 | HCC | CUGAAGUGAUGUGUAACUGAUCAG | SEQ ID NO 8 |
| hsa-miR-645 | 0.039255172 | HCC | UCUAGGCUGGUACUGCUGA | SEQ ID NO 11 |
| hsa-miR-641 | 0.039353 | HCC | AAAGACAUAGGAUAGAGUCACCUC | SEQ ID NO 17 |
| hsa-miR-19b-1* | 0.04526782 | HCC | AGUUUUGCAGGUUUGCAUCCAGC | SEQ ID NO 2 |
| | | | | |
| hsa-miR-93* | 0.04676072 | HD | ACUGCUGAGCUAGCACUUCCCG | SEQ ID NO 62 |
| hsa-miR-186 | 0.04824365 | HD | CAAAGAAUUCUCCUUUUGGGCU | SEQ ID NO 58 |

In particular, Table 2 shows the miRNAs present in a higher quantity in the samples from subjects affected by hepatocellular carcinoma versus the healthy controls:

**Table 2:**

| **MicroRNA (miRNA) Name** | **MicroRNA (miRNA) Sequence** | **MiRNA Sequence Number** |
|---|---|---|
| hsa-miR-223* | CGUGUAUUUGACAAGCUGAGUU | SEQ ID NO 1 |
| hsa-miR-19b-1* | AGUUUUGCAGGUUUGCAUCCAGC | SEQ ID NO 2 |
| hsa-miR-188-5p | CAUCCCUUGCAUGGUGGAGGG | SEQ ID NO 3 |
| hsa-miR-99b* | CAAGCUCGUGUCUGUGGGUCCG | SEQ ID NO 4 |
| hsa-miR-509-3p | UGAUUGGUACGUCUGUGGGUAG | SEQ ID NO 5 |
| hsa-miR-760 | CGGCUCUGGGUCUGUGGGGA | SEQ ID NO 6 |
| hsa-miR-520c-3p | AAAGUGCUUCCUUUUAGAGGGU | SEQ ID NO 7 |
| hsa-miR-573 | CUGAAGUGAUGUGUAACUGAUCA G | SEQ ID NO 8 |
| hsa-miR-877 | GUAGAGGAGAUGGCGCAGGG | SEQ ID NO 9 |
| hsa-miR-643 | ACUUGUAUGCUAGCUCAGGUAG | SEQ ID NO 10 |
| hsa-miR-645 | UCUAGGCUGGUACUGCUGA | SEQ ID NO 11 |
| hsa-miR-559 | UAAAGUAAAUAUGCACCAAAA | SEQ ID NO 12 |
| hsa-miR-571 | UGAGUUGGCCAUCUGAGUGAG | SEQ ID NO 13 |
| hsa-miR-122 | UGGAGUGUGACAAUGGUGUUUG | SEQ ID NO 14 |
| hsa-miR-885-5p | UCCAUUACACUACCCUGCCUCU | SEQ ID NO 15 |
| hsa-miR-483-5p | AAGACGGGAGGAAAGAAGGGAG | SEQ ID NO 16 |
| hsa-miR-641 | AAAGACAUAGGAUAGAGUCACCU C | SEQ ID NO 17 |
| hsa-miR-375 | UUUGUUCGUUCGGCUCGCGUGA | SEQ ID NO 18 |
| hsa-miR-567 | AGUAUGUUCUUCCAGGACAGAAC | SEQ ID NO 19 |

In particular, Table 3 shows the miRNAs present in lower quantity in the samples from subjects affected hepatocellular carcinoma versus the healthy controls:

**Table 3:**

| **MicroRNA (miRNA) Name** | **MicroRNA (miRNA) Sequence** | **miRNA Sequence Number** |
|---|---|---|
| hsa-miR-18b | UAAGGUGCAUCUAGUGCAGUUAG | SEQ ID NO 20 |
| hsa-miR-628-5p | AUGCUGACAUAUUUACUAGAGG | SEQ ID NO 21 |
| hsa-miR-107 | AGCAGCAUUGUACAGGGCUAUCA | SEQ ID NO 22 |
| hsa-miR-23a | AUCACAUUGCCAGGGAUUUCC | SEQ ID NO 23 |
| hsa-miR-210 | CUGUGCGUGUGACAGCGGCUGA | SEQ ID NO 24 |
| hsa-miR-1 | UGGAAUGUAAAGAAGUAUGUAU | SEQ ID NO 25 |
| hsa-miR-377 | AUCACACAAAGGCAACUUUUGU | SEQ ID NO 26 |
| hsa-miR-579 | UUCAUUUGGUAUAAACCGCGAUU | SEQ ID NO 27 |
| hsa-miR-223 | UGUCAGUUUGUCAAAUACCCCA | SEQ ID NO 28 |
| hsa-miR-145* | GGAUUCCUGGAAAUACUGUUCU | SEQ ID NO 29 |
| hsa-miR-331-5p | CUAGGUAUGGUCCCAGGGAUCC | SEQ ID NO 30 |
| hsa-miR-140-5p | CAGUGGUUUUACCCUAUGGUAG | SEQ ID NO 31 |
| hsa-miR-532-3p | CCUCCCACACCCAAGGCUUGCA | SEQ ID NO 32 |
| hsa-miR-16 | UAGCAGCACGUAAAUAUUGGCG | SEQ ID NO 33 |
| hsa-miR-494 | UGAAACAUACACGGGAAACCUC | SEQ ID NO 34 |
| hsa-miR-132 | UAACAGUCUACAGCCAUGGUCG | SEQ ID NO 35 |
| hsa-miR-29a | UAGCACCAUCUGAAAUCGGUUA | SEQ ID NO 36 |
| hsa-miR-590-5p | GAGCUUAUUCAUAAAAGUGCAG | SEQ ID NO 37 |
| hsa-miR-195 | UAGCAGCACAGAAAUAUUGGC | SEQ ID NO 38 |
| hsa-miR-197 | UUCACCACCUUCUCCACCCAGC | SEQ ID NO 39 |
| hsa-miR-193a-5p | UGGGUCUUUGCGGGCGAGAUGA | SEQ ID NO 40 |
| hsa-miR-24 | UGGCUCAGUUCAGCAGGAACAG | SEQ ID NO 41 |
| hsa-miR-140-3p | UACCACAGGGUAGAACCACGG | SEQ ID NO 42 |
| hsa-miR-19b | UGUGCAAAUCCAUGCAAAACUGA | SEQ ID NO 43 |
| hsa-miR-19a | UGUGCAAAUCUAUGCAAAACUGA | SEQ ID NO 44 |
| hsa-miR-222 | AGCUACAUCUGGCUACUGGGU | SEQ ID NO 45 |
| hsa-miR-93 | CAAAGUGCUGUUCGUGCAGGUAG | SEQ ID NO 46 |
| hsa-miR-15b | UAGCAGCACAUCAUGGUUUACA | SEQ ID NO 47 |
| hsa-miR-25 | CAUUGCACUUGUCUCGGUCUGA | SEQ ID NO 48 |
| hsa-miR-655 | AUAAUACAUGGUUAACCUCUUU | SEQ ID NO 49 |
| hsa-miR-365 | UAAUGCCCCUAAAAAUCCUUAU | SEQ ID NO 50 |
| hsa-miR-199a-3p | ACAGUAGUCUGCACAUUGGUUA | SEQ ID NO 51 |
| hsa-miR-625* | GACUAUAGAACUUUCCCCCUCA | SEQ ID NO 52 |
| hsa-miR-142-3p | UGUAGUGUUUCCUACUUUAUGGA | SEQ ID NO 53 |
| hsa-miR-454 | UAGUGCAAUAUUGCUUAUAGGGU | SEQ ID NO 54 |
| hsa-miR-345 | GCUGACUCCUAGUCCAGGGCUC | SEQ ID NO 55 |
| | | |
| hsa-let-7b | UGAGGUAGUAGGUUGUGUGGUU | SEQ ID NO 56 |
| hsa-miR-20a | UAAAGUGCUUAUAGUGCAGGUAG | SEQ ID NO 57 |
| hsa-miR-186 | CAAAGAAUUCUCCUUUUGGGCU | SEQ ID NO 58 |
| hsa-miR-191 | CAACGGAAUCCCAAAAGCAGCUG | SEQ ID NO 59 |
| hsa-miR-451 | AAACCGUUACCAUUACUGAGUU | SEQ ID NO 60 |
| hsa-let-7g | UGAGGUAGUAGUUUGUACAGUU | SEQ ID NO 61 |
| hsa-miR-93* | ACUGCUGAGCUAGCACUUCCCG | SEQ ID NO 62 |

Figure 1 shows the values of the relative miRNA expression ratio between samples from subjects affected by hepatocellular carcinoma (HCC) and samples from healthy control subjects (HD) . Values between 0 and 1 indicate overexpressed miRNAs in the healthy samples, whereas values greater than 1 refer to overexpressed miRNAs in the hepatocellular carcinoma samples. Figure 2 shows a graphical representation, by colour gradient (heatmap), of the ΔCt values for the 62 miRNAs differentially expressed, in a significant manner according to the t-test analysis, between unpaired samples, 10 from healthy subjects and 10 from subjects with hepatocellular carcinoma HCC (p-value <0.05). The gradient value goes from white (maximum expression, low ΔCt value) to black (minimum expression, maximum ΔCt value). Columns: the 20 samples considered, 10 samples from healthy donors (code "DctHD#") and 10 from donors affected by hepatocellular carcinoma (code "DctHCC#") where "#" indicates the sample ID. Rows: the 62 significant miRNAs with corresponding standard names.

Similarity relations between samples and between miRNAs based on their expression values are graphically expressed by the dendrograms in Figure 2, calculated in an automatic (unsupervised) manner using the Euclidean distance method.

### EXAMPLE 2

The presence of miRNAs was analysed in the peripheral blood of five subjects with hepatocellular carcinoma and the peripheral blood of the same subjects prior to the development of hepatocellular carcinoma, when they showed a clinical profile of cirrhosis, was used as the control.

A "quantitative RT PCR" analysis conducted and analysed as illustrated in example 1 showed the presence of 11 miRNAs, described in Table 4, which were present in a higher or lower quantity in subjects with hepatocellular carcinoma versus the controls.

**Table 4 :**

| **miRNA** | **raw p-val** | **higher in** | **Target Sequence** | **miRNA Sequence Number** |
|---|---|---|---|---|
| hsa-miR-599 | 0.0032 | HCC | GUUGUGUCAGUUUAUCAAAC | SEQ ID NO 69 |
| hsa-miR-485-3p | 0.0049 | HCC | GUCAUACACGGCUCUCCUCUCU | SEQ ID NO 64 |
| hsa-miR-29a | 0.0057 | CIRR | UAGCACCAUCUGAAAUCGGUUA | SEQ ID NO 36 |
| hsa-miR-23a | 0.0184 | CIRR | AUCACAUUGCCAGGGAUUUCC | SEQ ID NO 23 |
| hsa-miR-193a-5p | 0.0202 | CIRR | UGGGUCUUUGCGGGCGAGAUGA | SEQ ID NO 40 |
| hsa-miR-299-3p | 0.0248 | HCC | UAUGUGGGAUGGUAAACCGCUU | SEQ ID NO 65 |
| hsa-miR-138-1* | 0.0272 | HCC | GCUACUUCACAACACCAGGGCC | SEQ ID NO 63 |
| hsa-miR-519d | 0.0292 | CIRR | CAAAGUGCCUCCCUUUAGAGUG | SEQ ID NO 67 |
| hsa-miR-133b | 0.0306 | HCC | UUUGGUCCCCUUCAACCAGCUA | SEQ ID NO 66 |
| hsa-miR-760 | 0.0347 | HCC | CGGCUCUGGGUCUGUGGGGA | SEQ ID NO 6 |
| hsa-miR-190b | 0.0352 | CIRR | UGAUAUGUUUGAUAUUGGGUU | SEQ ID NO 68 |

In particular, the miRNAs shown in Table 5 were present in a higher quantity in the samples from subjects affected by hepatocellular carcinoma versus the cirrhotic subjects (controls) :

**Table 5:**

| **MicroRNA (miRNA)** | **MicroRNA (miRNA) Sequence** | **MicroRNA (miRNA) Sequence Number** |
|---|---|---|
| hsa-miR-138-1* | GCUACUUCACAACACCAGGGCC | SEQ ID NO 63 |
| hsa-miR-760 | CGGCUCUGGGUCUGUGGGGA | SEQ ID NO 6 |
| hsa-miR-599 | GUUGUGUCAGUUUAUCAAAC | SEQ ID NO 69 |
| hsa-miR-485-3p | GUCAUACACGGCUCUCCUCUCU | SEQ ID NO 64 |
| hsa-miR-299-3p | UAUGUGGGAUGGUAAACCGCUU | SEQ ID NO 65 |
| hsa-miR-133b | UUUGGUCCCCUUCAACCAGCUA | SEQ ID NO 66 |

In particular, the miRNAs shown in Table 6 were present in a lower quantity in the samples from subjects affected by hepatocellular carcinoma versus cirrhotic subjects (controls):

**Table 6:**

| **MicroRNA (miRNA) Name** | **MicroRNA (miRNA) Sequence** | **MicroRNA (miRNA) Sequence Number** |
|---|---|---|
| hsa-miR-23a | AUCACAUUGCCAGGGAUUUCC | SEQ ID NO 23 |
| hsa-miR-193a-5p | UGGGUCUUUGCGGGCGAGAUGA | SEQ ID NO 40 |
| hsa-miR-519d | CAAAGUGCCUCCCUUUAGAGUG | SEQ ID NO 67 |
| hsa-miR-29a | UAGCACCAUCUGAAAUCGGUUA | SEQ ID NO 36 |
| hsa-miR-190b | UGAUAUGUUUGAUAUUGGGUU | SEQ ID NO 68 |

Figure 3 shows the values of the relative miRNA expression ratio differentially expressed in cirrhotic subjects before and after the appearance of hepatocellular carcinoma. Values between 0 and 1 indicate overexpressed miRNAs in the healthy samples, whereas values greater than 1 refer to overexpressed miRNAs in the samples from subjects affected by hepatocellular carcinoma.

Figure 4 shows a graphical representation, by colour gradient (heatmap), of the ΔCt values for the 11 miRNAs differentially expressed, in a significant manner according to the t-test analysis, between 5 blood samples from subjects affected by liver cirrhosis and 5 blood samples from the same cirrhotic subjects, who subsequently developed hepatocellular carcinoma.

The gradient value goes from white (maximum expression, low ΔCt value) to black (minimum expression, maximum ΔCt value). Columns: the 10 samples considered, 5 cirrhotic samples (code "Dctcirr#") and 5 hepatocellular carcinoma samples (code "DctHCC#"), where "#" indicates the sample ID. Rows: the 11 significant miRNAs with corresponding standard names.

Similarity relations between samples and between miRNAs, based on their expression values, are graphically expressed by the dendrograms in Figure 4, calculated in an automatic (unsupervised) manner using the Euclidean distance method.

## Claims

1. A method for diagnosing or prognosticating hepatocellular carcinoma, also in the early stages, or for assessing the risk of developing hepatocellular carcinoma, or for monitoring the effectiveness of an anti-tumour therapy against hepatocellular carcinoma, comprising the step of measuring, in an isolated sample of peripheral blood or biological fluid, the expression level of miRNA gene products consisting of at least of miR-885-5p of SEQ ID No 15, as compared to a reference expression level.

2. The method according to claim 1, wherein the miRNA gene products further comprise at least one chosen from the group consisting of SEQ ID NO 1-14, 16-19, SEQ ID NO 63-66 and SEQ ID 69, as compared to a reference expression level.

3. The method according to claim 1 or 2, wherein the miRNA gene products further comprise at least one chosen from the group consisting of SEQ ID NO 20-62 and SEQ ID NO 67-68, as compared to a reference expression level.

4. The method according to claim 2 or 3, wherein the miRNA gene products are chosen from the group consisting of SEQ ID NO 6, 9, 12, 13, 14, 16 and 65, and 23, 28, 30, 31, 33, 36, 37, 39, 40, 41 and 67, as compared to a reference expression level.

5. The method according to claim 1 to 4, wherein said peripheral blood sample is chosen from among whole blood, blood peripheral mononucleated cells, serum or plasma; said biological fluid sample is chosen between urine or saliva.

6. The method according to any of the claims 1 to 5, wherein said method for monitoring the effectiveness of an anti-tumour therapeutic treatment comprises measuring the alteration in the expression levels of at least one miRNA gene product in a sample of the test subject, as compared to a sample of the same subject in different phases of the anti-tumour therapeutic treatment.

7. The method according to any of the claims 1 to 5, wherein said method for monitoring the effectiveness of an anti-tumour therapeutic treatment comprises comparing peripheral blood samples from patients affected by hepatocellular carcinoma who are undergoing an anti- tumour therapeutic treatment and samples from patients affected by hepatocellular carcinoma who are not undergoing an anti-tumour therapeutic treatment and measuring the alteration in the expression levels of a miRNA gene product between the two groups of patients.

8. The method according to any of the claims 1 to 7, wherein said method is a method for diagnosing or assessing the risk of developing liver cirrhosis, or for prognosticating the evolution of liver cirrhosis in patients affected by cirrhosis, or for monitoring the effectiveness of a pharmacological therapy against liver cirrhosis.

9. A use of the method according to any of the claims 1 to 8 to identify new therapeutic targets.

10. A pharmaceutical composition comprising a pharmaceutically acceptable carrier and at least one isolated miRNA gene product according to claim 1 or 2 and/or a nucleic acid complementary thereto.

11. The composition according to claim 10 for use in the treatment of hepatocellular carcinoma or for the treatment of liver cirrhosis.
